# EUROPEAN PATENT SPECIFICATION

(11) **EP 1 691 783 B1**
(45) Date of publication and mention of the grant of the patent: **25.11.2009**
(21) Application number: 04801711.5
(22) Date of filing: 02.12.2004
(51) Int. Cl.: A61K 9/14, A61K 9/72, A61M 15/00, A61J 1/00, A61K 31/46, A61P 11/06, A61P 11/08

(54) **PRE-METERED DRY POWDER INHALER FOR MOISTURE-SENSITIVE MEDICAMENTS**
VORDOSIERTER TROCKENPULVERINHALATOR FÜR FEUCHTIGKEITSEMPFINDLICHE MEDIKAMENTE
POUDRE SECHE PREDOSEE A INHALER POUR MEDICAMENTS HYDROSENSIBLES

(30) Priority: 03.12.2003 SE 0303269; 22.12.2003 SE 0303569; 03.09.2004 US 933219
(43) Date of publication of application: 23.08.2006
(73) Proprietor: Boehringer Ingelheim International GmbH, 55216 Ingelheim am Rhein (DE)
(72) Inventor: MYRMAN, Mattias, S-13542 Tyreso (SE); CALANDER, Sven, S-645 32 Strängnäs (SE); NIEMI, Alf, S-645 50 Strängnäs (SE)
(74) Representative: Eckhardt, Conrad
(86) International application number: PCT/SE2004/001794
(87) International publication number: WO 2005/053648

(56) References cited:
- WO-A1-01/27210
- WO-A1-02/24266
- WO-A1-02/30389
- WO-A1-03/006092
- WO-A1-03/066437
- WO-A1-03/084502
- WO-A1-03/086517
- WO-A2-00/74754
- WO-A2-02/00280
- US-A1- 2002 053 344
- US-A1- 2003 136 405

## Description

The present invention relates to a dry powder inhaler (DPI) delivering a high and stable fine particle dose. The inhaler utilizes a high barrier seal container filled with at least one metered dose of a formulation comprising at least one excipient and a tiotropium medicament.

Additional advantages and other features of the present invention will be set forth in part in the description that follows and in part will become apparent to those having ordinary skill in the art upon examination of the following or may be learned from the practice of the present invention. The advantages of the present invention may be realized and obtained as particularly pointed out in the appended claims. As will be realized, the present invention is capable of other and different embodiments, and its several details are capable of modifications in various obvious respects, all without departing from the present invention. The description is to be regarded as illustrative in nature, and not as restrictive.

### BACKGROUND OF THE INVENTION

Dry powder inhalers (DPI) are becoming more and more popular because of their ease of use and medical efficacy. DPI's can be divided into two major categories: bulk and pre-metered devices. Pre-metered devices are gaining more and more market share due to the ability to control the product and process of metering a correct dose to the user. DPIs with pre-metered doses are, because of this, more reliable than bulk inhalers that meter the powder dose inside the inhaler. A pre-metered DPI moves the critical step of metering a dose to a pharmaceutical production process.

Asthma and chronic obstructive pulmonary disease (COPD) affect more than 30 million people in the United States. More than 100,000 deaths each year are attributable to these conditions. Obstruction to airflow through the lungs is the characteristic feature in each of these airway diseases, and the medications utilized in treatment are often similar. Chronic obstructive pulmonary disease (COPD) is a widespread chronic lung disorder encompassing chronic bronchitis and emphysema. The causes of COPD are not fully understood. Experience shows that the most important cause of chronic bronchitis and emphysema is cigarette smoking. Air pollution and occupational exposures may also play a role, especially when combined with cigarette smoking. Heredity also causes some emphysema cases, due to alphal anti-trypsin deficiency.

Administration of asthma drugs by an oral inhalation route is very much in focus today, because of advantages offered like rapid and predictable onset of action, cost effectiveness and high level of comfort for the user. Dry powder inhalers (DPI) are especially interesting as an administration tool, compared to other inhalers, because of the flexibility they offer in terms of nominal dose range, i. e. the amount of active substance that can be administered in a single inhalation.

Tiotropium, and especially the bromide salts thereof, is an effective bronchodilator. Tiotropium has a relatively fast onset and a long duration of action, which may last for 24 hours or longer. Tiotropium reduces the vagal cholinergic tone of the smooth muscle, which is the main reversible component of COPD. Tiotropium has been shown to cause quite insignificant side effects in clinical testing, dryness of mouth and constipation being perhaps the most common symptoms. Because it is often very difficult to diagnose asthma and COPD correctly and since both disorders may co-exist, it is advantageous to treat patients suffering temporary or continuous bronchial obstruction resulting in dyspnoea with a small but efficient dose of a long-acting tiotropium, preferably tiotropium bromide, because of its fast onset, long duration and small adverse side effects. Today, a bronchodilating medicament like tiotropium is often co-prescribed and administered in combination with other asthma medicaments in order to provide a combined therapy, e. g. combining a bronchodilating and an anti-inflammatory treatment.

Methods for the administration of tiotropium bromide containing inhalation powders are for instance described in WO 03/084502, which discloses in figure 1 the capsule inhaler that is used for the COPD therapy with tiotropium bromide. In this inhaler capsules containing the tiotropium containing inhalation powder are placed in the inhaler, pierced and the powder is thus set free for inhalation.

Dose efficacy depends to a great deal on delivering a stable and high fine particle dose (FPD) out of the dry powder inhaler. The FPD is the respirable dose mass out of the dry powder inhaler with an aerodynamic particle size below 5 µm. Thus, when inhaling a dose of any kind of dry medication powder it is important to obtain by mass a high fine particle fraction (FPF) of particles with an aerodynamic size preferably less than 5 µm in the inspiration air. The majority of larger particles (>5 µm) does not follow the stream of air into the many bifurcations of the airways, but get stuck in the throat and upper airways, where the medicament is not giving its intended effect, but may instead be harmful to the user. It is also important to keep the dosage to the user as exact as possible, to maintain a stable efficacy over time, and that the medicament dose does not deteriorate during normal storage. For instance, Boehringer Ingelheim KG (BI) markets tiotropium bromide under the proprietary name of Spiriva^{®}. Surprisingly, in a recent investigation into the Spiriva^{®} product we have found that the Spiriva^{®}/HandiHaler^{®} system from BI for administration by inhalation of doses contained in gelatin capsules shows poor performance and has short in-use stability.

There are several prior art methods, applicable to tiotropium, of manufacturing medicament formulations suitable for inhalation by a dry powder inhaler device. In one such method tiotropium and an excipient are suspended in a liquid and then stirred and after obtaining a mixture the liquid is evaporated. Mixing substances with different particle sizes is another method, which teaches how to manufacture a uniform powder blend by a special mixing procedure. Yet another method teaches how to carry out a continuous dosing into a mixer to obtain a uniform powder formulation. Further methods, which may be used to produce a uniform powder formulation of the excipient or excipents and the tiotropium substance encompasses using air or some other pharmaceutically acceptable gas as a suspending medium in a batch or continuous mixing process to prepare a uniform mixing of the particles of excipient(s) and tiotropium and optionally one or more additional pharmacologically active ingredients (API).

Preparing a formulation of tiotropium and an excipient where the amount of tiotropium is very small (e.g., < 1:100 the amount of the excipient) is of utmost importance for the FPD. Several prior art methods are aimed at improved preparation of excipients in order to improve the active ingredient FPD e.g. coating the excipient to present a fluorinated particle surface. Other surface modifications and surface treatment methods are possible to use to improve the FPD performance of the formulation.

It is not uncommon in the prior art to incorporate a desiccant into the material of the container or into the device or into the outer package for the device. The amount of desiccant is normally very small in this type of construction and the demands on the container seal to protect the medicament powder remains the same if the desiccant is not to be destroyed before opening of the product.

Methods of dose forming of tiotropium formulations include conventional mass, gravimetric or volumetric metering and devices and machine equipment well known to the pharmaceutical industry for filling blister packs, for example. Also see WO 03/27617 A1, WO 03/66437 A1, WO 03/66436 A1, WO 03/26965 A1, WO 02/44669 A1 and DE 100 46 127 A1, DE 202 09 156 U1 for examples of prior art in volumetric and/or mass methods and devices for producing doses of medicaments in powder form. Electrostatic forming methods may also be used, for example as disclosed in US 6,007,630 and US 5,699,649.

A most suitable method of depositing microgram and milligram quantities of dry powders uses electric field technology (ELFID) as disclosed in our U.S. Patent No. 6,592,930 B2, which is hereby incorporated in this document in its entirety as a reference. In this method powder flowability is unimportant, because powder particles are transported from a bulk source to a dose bed in a dose-forming step, not relying on the force of gravity but using primarily electric and electrostatic force technology to deposit a metered quantity of powder, i.e. a dose, onto the dose bed, which may be a blister, capsule or high barrier container as disclosed in the present invention. An advantage of this electric field dose forming process is that it is not necessary to add large excipient particles to the medicament powder, because good powder flowability is not an issue. Excipients are added to the active agent, particularly tiotropium, in order to dilute the drug to have a pre-metered dose in the inhaler exceeding 100 µg. Advantageously, the excipient is finely divided so that the mass median aerodynamic diameter (MMAD) is less than 10 µm. Tests confirm that the fine particle dose (FPD) from a dose formed by the electric field method is considerably better than the FPD from a similar dose formed by other methods common in prior art. The electric field method is also very suitable for combined doses, such as tiotropium mixed with APIs or separately forming and depositing metered quantities of the active medicaments in the same container.

Dry powder inhalers using peelable foils for in-use dose protection are known in prior art. The peelable lid foil is made out of a laminate with heat seal laquer (HSL) sealing to the PVC layer of the base laminate after the powder is filled into a formed cavity in the base laminate. The process of filling is very important, because any powder left on the heat sealable surfaces will very negatively affect the quality of the seal. A peelable HSL is always much more sensitive and difficult to seal compared to conventional sealing foils. It is often necessary to have an external high barrier package to preserve the inhaler for the shelf-life period and have the peelable HSL to protect the powder during the in-use time only. This type of prior art inhaler opens the powder dose before the inhaler is ready for inhalation and the dose is thereby exposed to the surrounding environment and the possible exhalation moist air of the user.

An objective of the present invention is the preservation and delivery of a high fine particle dose (FPD) of tiotropium by a DPI product comprising a metered dose of tiotropium medicament, adapted for inhalation, packaged in a dry and tight container, such that the FPD when delivered is unaffected for the shelf life of the medical product by normal variations in ambient conditions during handling, storage and delivery using the DPI product. As will become apparent below, the inventors have met this objective, and more.

### SUMMARY OF THE INVENTION

The present invention discloses a DPI product adapted for use in the treatment of respiratory disorders, and comprises a pre-metered dry powder medicament, which includes at least one excipient, tiotropium and optionally at least one further active pharmaceutical ingredient (API). Furthermore, the dose in the DPI is directly metered, loaded and sealed into a moisture-tight, dry container acting as a dry, high barrier seal against moisture.

The invention DPI comprises a pre-metered dry powder dose having a high FPD, and enables the selection of suitable qualified excipients for good moisture properties and the forming of doses achieving a high FPD (e. g., from both an electrical field dosing technology standpoint and from conventional volumetric filling methods) In a different aspect of the invention one or more excipients are included in selected ratios with tiotropium in a dry powder formulation, such that the functions of the excipient or excipients are, inter alia, to dilute the potent tiotropium ingredient and/or to make the flowability of the dry powder formulation acceptable for the dose forming process and/or to optimize the FPD of the metered dose.

In another aspect of the invention a type of inhaler is disclosed, which can accept at least one sealed, moisture-tight, dry container enclosing a metered tiotropium dose and deliver said dose with a consistent FPD, over the expected shelf life of the product.

In a further aspect of the invention tiotropium is mixed or formulated with one or more additional, pharmacologically active ingredient (s) (API) thereby combining the tiotropium medicament with other medicament (s) to be used in the treatment of respiratory disorders. The present invention encompasses such use of tiotropium in a combined dose of medicaments in stable formulations, which are directly metered and loaded into a sealed, moisture-tight, dry container for insertion into a DPI, the combined dose adapted for inhalation by the user.

Further, the invention discloses a method of preventing moisturized air from a user to reach the powder in the dose prior to an inhalation and still further a method of making the dose available for aerosolizing in the same moment, as the seal to the container enclosing the dose is broken.

### BRIEF DESCRIPTION OF THE DRAWINGS

The invention, together with further objects and advantages thereof, may best be understood by referring to the following detailed description taken together with the accompanying drawings, in which:
- FIG. 1: illustrates in a graph the results of tests S1 to S5 and HBS1 to HBS3;
- FIG. 2: illustrates sorption properties of pharmaceutical excipients;

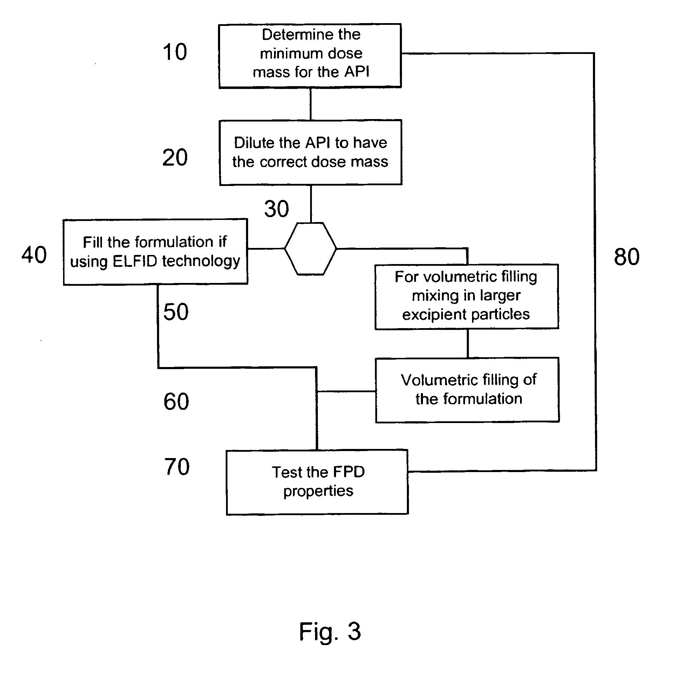

- FIG.3: illustrates in a flow-chart a method of developing a pharmaceutical composition with high FPD;
- FIG. 4: illustrates in top and side views a first embodiment of a dose deposited onto a dose bed and a high barrier seal, and
- FIG. 5: illustrates in top and side views a second embodiment of a dose onto a dose bed and a high barrier seal.

### DETAILED DESCRIPTION OF THE PREFERRED EMBODIMENTS

The present invention relates to a DPI loaded with a moisture sensitive drug or drugs, comprising tiotropium, and describes doses and dose delivery for achieving high scores of delivered FPD. Preferably, the DPI is pre- metered. Further, the invention solves the problem of how such sensitive drugs can be protected from moisture from the moment doses are formed and sealed to the moment a user inhales a selected dose, through all stages of storing, transporting, distributing, again storing and finally using a dose.

Further, suitable dry powder inhalers for moisture sensitive dosages are disclosed.

The present invention discloses a dry, moisture-tight, directly loaded and sealed container enclosing a metered dose of tiotropium in a high FPD formulation containing at least one excipient. The term "tiotropium" is a generic term for all active forms thereof, including pharmaceutically acceptable salts (particularly bromide), derivates, hydrates, solvates or mixtures thereof. A metered dose normally includes at least one excipient. The container uses dry, high barrier seals impervious to moisture and other foreign matter that comprise aluminium and is adapted for insertion into a dry powder inhaler device or the container may be adapted to be a part of an inhaler device.

"Dry"means that the walls of the container are constructed from selected materials such that the walls, especially the inside wall surface of the container, cannot release water that may affect the anticholinergic drug powder in the dose such that the FPD is reduced. As a logical consequence container construction and materials should not be in need of processes suggested in the German publication DE 101 26 924 A1. As an example, gelatin is not a dry material and even after a special drying process gelatin still contains water.

"High barrier seal"means a dry packaging construction or material or combinations of materials comprising aluminium. A high barrier seal is wherein it represents a high barrier against moisture and that the seal itself is'dry', i.e. it cannot give off measurable amounts of water to the load of powder. A high barrier seal may for instance be made up of one or more layers of materials, i.e. technical polymers, aluminum or other metals, glass, siliconoxides etc that together constitutes the high barrier seal. If the high barrier seal is a foil, a 50 □m PCTFE/PVC pharmaceutical foil is the minimum required high barrier foil if a two week in-use stability should be achieved. For longer in-use stabilities metal foils like aluminum foils from Alcan Singen can be used.

A "high barrier container" is a mechanical construction made to harbor and enclose a dose of e.g. tiotropium. The high barrier container is built using high barrier seals constituting the walls of the container.

"Directly loaded" means that the metered dose is loaded directly into the high barrier container, i.e. without first loading the dose into e.g. a gelatin capsule, and then enclosing one or more of the primary containers (capsules) in a secondary package made of a high barrier seal material.

Tiotropium is an excellent bronchodilating medicament because it has a fast onset and it is long-acting, even longer than 24 hours, which makes it ideal for many asthmatics. It is a potent drug and a once daily administration by inhalation is sufficient to manage asthma. If the user suffers an acute attack of asthma, then an extra administration of the tiotropium drug brings the asthma attack under control again. But tiotropium is extremely sensitive to moisture. This fact is e.g. documented in the report 'COLLEGE TER BEOORDELING VAN GENEESMIDDELEN MEDICINES EVALUATION BOARD; PUBLIC ASSESSMENT REPORT; Spiriva 18 µg, inhalation powder in hard capsules; RVG 26191' (2002-05-21) on page 6/28 under 'Product development and finished product' a very short in-use stability of the Spiriva^{®} product (9 days) is reported and a brittleness of the capsule in the blister pack and a very low FPD: 'about 3 ug'. The capsules are packed in a blister made of polyvinylchloride and a protective aluminium layer. One blistercard consists of two 5-cavity blisters joined along a perforated line. An aluminum peel-off foil covers the cavities. The blister allows taking one capsule at a time, so the other capsules remain protected from moist air.

This polyvinylchloride film is evidently not adequate to protect SPIRIVA^{®} capsules for more than 9 days in an in-use situation.

Details about a prior art inhalation kit comprising inhalable powder of tiotropium and use of an inhaler for the administration of tiotropium may also be studied in the international publication WO 03/084502 A1. Details about tiotropium compounds, medicaments based on such compounds, the use of compounds and processes for preparing compounds may be studied in the European Patent Application 0418716B1.

In the light of the above information given in the quoted report a program was set up for a stability-test of the Spiriva^{□} product according to Food and Drug Administration (FDA) recommendations.

Spiriva^{®} is administered by the HandiHaler^{®} DPI. Spiriva^{®} is a formulation of tiotropium and a finely divided excipient and a larger excipient for volumetric filling into a gelatine capsule that is dried down after filling and then packaged into a tropical blister made of PVC foil. The blister is then covered with an aluminium foil. During the in-use time after opening the first capsule only the PVC foil protects the remaining 4 capsules in the blister.

A 3 week test program in accelerated conditions (40 ± 2 °/ 75 ± 5 RH) for the container closure of the Spiriva^{®} product, in this case the capsule and the blister pack, and the impact of the capsule and the blister package on the FPD was set up and tested.

### Execution of tests

Spiriva^{®} powder formulation in bulk and Spiriva^{®} capsules from our local pharmacy were introduced to the laboratory together with the HandiHaler^{®}. The laboratory was set up to perform in-vitro tests according to European Pharmacopoeia (EP) and US Pharmacopoeia (USP) using two Andersen cascade impactors. All analytical work where then performed according to standardized methods for Physical Tests and Determinations for Aerosols, metered-dose inhalers and dry powder inhalers described in pharmacopoeias (e.g. USP 2002 <601>) using a state of the art High Performance Liquid Chromatograph (HPLC) system.

### Spiriva^{®} tests

### Test S1

Aerodynamic fine particle fraction of metered and delivered dose out of Handihaler^{®} using Spiriva^{®} formulation from bulk powder loaded into originator capsules during relative humidity below 10 %. The test was performed with 4 kPa pressure drop over the HandiHaler^{®} at room temperature and laboratory ambient conditions.

### Test S2

Aerodynamic fine particle fraction of metered and delivered dose out of Handihaler^{®} using commercial Spiriva^{®} capsules purchased from our local pharmacy. Test performed with 4 kPa pressure drop over the HandiHaler^{®} at room temperature and laboratory ambient conditions.

### Test S3

An in-use stability test of the aerodynamic fine particle fraction of metered and delivered dose out of Handihaler^{®} using commercial Spiriva^{®} capsules purchased from our local pharmacy. From the blister holding 5 capsules one capsule was withdrawn and the remaining 4 capsules were put 4 days into 40 °C and 75 % Rh. The blister containing the 4 capsules was then put in an exicator for 2 h before tests were performed. The test was performed with 4 kPa pressure drop over the HandiHaler^{®} at room temperature and laboratory ambient conditions.

### Test S4

An in-use stability test of the aerodynamic fine particle fraction of metered and delivered dose out of Handihaler^{®} using commercial Spiriva^{®} capsules purchased from our local pharmacy. From the blister holding 5 capsules one capsule was withdrawn and the remaining 4 capsules were put 13 days into 40 °C and 75 % Rh. The blister containing the 4 capsules was then put in an exicator for 2 h before tests were performed. The test was performed with 4 kPa pressure drop over the HandiHaler^{®} at room temperature and laboratory ambient conditions.

### Test S5

An in-use stability test of the aerodynamic fine particle fraction of metered and delivered dose out of Handihaler^{®} using commercial Spiriva^{®} capsules purchased from our local pharmacy. From the blister holding 5 capsules one capsule was withdrawn and the remaining 4 capsules were put 21 days into 40 °C and 75 % Rh. The blister containing the 4 capsules was then put in an exicator for 2 h before tests were performed. The test was performed with 4 kPa pressure drop over the HandiHaler^{®} at room temperature and laboratory ambient conditions.

### High barrier seal tests

### Test HBS1

An in-use stability test of the aerodynamic fine particle fraction of metered and delivered dose out of Handihaler^{®} using Spiriva^{®} formulation from bulk powder loaded during relative humidity below 10 % into containers made to act as a high barrier seal, in this case aluminum foils from Alcan Singen Germany and then sealed to absolute tightness. The aluminum containers were put in an exicator for 2 h before the Spiriva^{®} powder formulation was loaded from the aluminum containers into the originator capsules at a relative humidity below 10 %. The test was performed with 4 kPa pressure drop over the HandiHaler^{®} at room temperature and laboratory ambient conditions.

### Test HBS2

An in-use stability test of the aerodynamic fine particle fraction of metered and delivered dose out of Handihaler^{®} using Spiriva^{®} formulation from bulk powder loaded during relative humidity below 10 % into containers made to act as a high barrier seal, in this case aluminum foils from Alcan Singen Germany and then sealed to absolute tightness. The sealed aluminum containers were put into climate chambers for 7 days at 40 °C and 75 % Rh. The aluminum containers were put in an exicator for 2 h before the Spiriva^{®} powder formulation was loaded from the aluminum containers into the originator capsules at a relative humidity below 10 %. The test was performed with 4 kPa pressure drop over the HandiHaler^{®} at room temperature and laboratory ambient conditions.

### Test HBS3

An in-use stability test of the aerodynamic fine particle fraction of metered and delivered dose out of Handihaler^{®} using Spiriva^{®} formulation from bulk powder loaded during relative humidity below 10 % into containers made to act as a high barrier seal, in this case aluminum foils from Alcan Singen Germany and then sealed to absolute tightness. The sealed aluminum containers were put into climate chambers for 14 days at 40 °C and 75 % Rh. The aluminum containers were then put in an exicator for 2 h before the Spiriva^{®} powder formulation was loaded from the aluminum containers into the originator capsules at a relative humidity below 10 %. The test was performed with 4 kPa pressure drop over the HandiHaler^{®} at room temperature and laboratory ambient conditions.

### C-haler DPI tests

A test was also made outside the stability test program to evaluate our proprietary pre-metered dry powder inhaler, the so-called C-haler, in comparison with the HandiHaler^{®}. The C-haler cartridge used high barrier seals made out of aluminum foils from Alcan Singen Germany and the containers where filled volumetrically with 5 mg of the Spiriva^{®} powder formulation in bulk. The test was performed using a 4 kPa pressure drop over the C-haler at room temperature and laboratory ambient conditions. The results from the Andersen impactor tests were calculated on fine particle fraction based on delivered dose as well as on metered dose and converted to FPD. The results are given in Table 1 below.

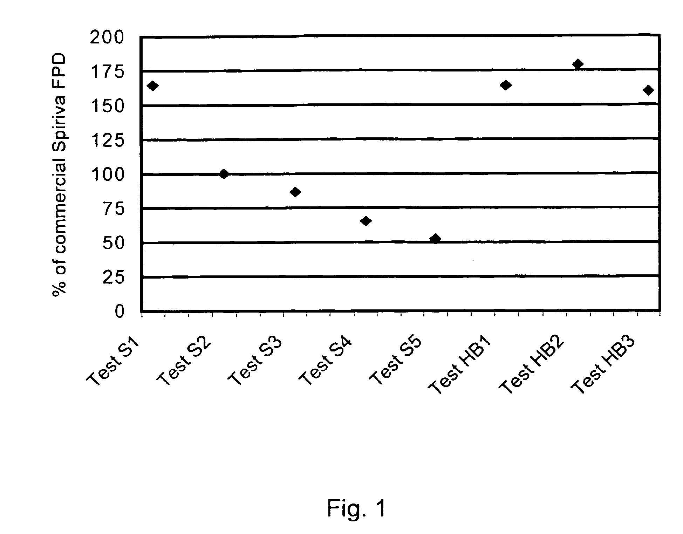

The results of tests S1-5 and HBS1-3 are plotted in Figure 1. The Y-axis is designated '% of commercial Spiriva^{®} FPD'. This relates to the FPD out from the Handihaler^{®}, where 100 % is the FPD from a fresh sample from the pharmacy.

**Table 1.**

| Inhaled fine particle dose (FPD) <5 □m in % | | |
|---|---|---|
| Calculation based on | Spiriva^{®} in HandiHaler^{®},Spiriva^{®} commercial sample, FPD | in C-haler, FPD |
| Metered dose | 18 % | 47 % |
| Delivered dose | 36 % | 56 % |

### Conclusion of the tests performed on Spiriva^{®}

Surprisingly we have found and concluded in our tests that the pre-metered doses of Spiriva^{®} are extremely sensitive to moisture and that a conventional packaging into gelatin capsules that are extensively used for inhalation products and especially respiratory products today, will seriously affect the FPD. The results show that there is a need for a dry, moisture-tight high barrier seal of the pre-metered dose enclosing the tiotropium formulation to preserve the original fine particle fraction and also that gelatin is not a proper excipient or material together with the Spiriva^{®} formulation inside a high barrier sealed container. Not so surprisingly in the light of these findings, we have also found that the tiotropium formulation must be properly protected also during the in-use time if further reduction of the FPD shall be avoided.

The tests carried out show that the moisture content of the gelatin capsule reduces the FPD out of the HandiHaler^{®} with approximately 50 % from the time of loading the dose into a capsule until the point in time when the product reaches the market. Loading Spiriva^{®} doses into dry containers made of materials presenting high barrier seal properties and then storing the loaded containers in 40 °C and 75 % Rh, before transferring the Spiriva^{®} doses to originator capsules and performing the same tests using HandiHaler^{®} as before, no change can be detected in the fine particle dose (FPD), even after long periods of time. The FPD of Spiriva^{®} in gelatin capsules, however, is further diminishing during the in-use time of the product and the FPD has been shown to drop up to another 20 % after 5 days of storage in 40 °C and 75 % Rh in an in-use stability test, due to the breaking of the moisture barrier of the blister package. Table 1 shows that our C-haler using high barrier containers shows a 2.6 times higher performance than HandiHaler^{®} with respect to FPD based on metered dose.

### State of the art

Metered doses of the Spiriva^{®} powder formulation are today at the originator manufacturing site loaded into gelatin capsules. A gelatin capsule contains typically 13-14 % water by weight in the dose forming stage and after the capsules have been loaded they are dried in a special process in order to minimize water content. A number of dried capsules are then put in a common blister package. Details about suitable state-of-the-art capsule materials and manufacturing processes may be studied in the German Patent Application DE 101 26 924 A1. The remaining quantity of water in the capsule material after drying is thus enclosed in the blister package. The equilibrium between the captured air inside the package and the gelatin capsule will generate a relative humidity inside the blister package that will negatively affect the FPD of tiotropium powder out of the dry powder inhaler.

It is interesting to note that the majority of dry powder formulations of many kinds of medicaments are not seriously affected by enclosed moisture in the capsule material or by normal storage variations in the relative humidity of the surrounding air. Examples of substances that are much more stable with respect to moisture are inhaled steroids e.g. budesonid and fluticasone. Surprisingly, our investigation has shown tiotropium to be very much different. By some as yet unknown mechanisms the FPD becomes less over time when affected by very small quantities of water. Since the capsules are only used as convenient, mechanical carriers of inter alia Spiriva^{®} doses, a solution in part to the moisture problem would be not to use capsules at all, but rather to directly load doses into containers made of dry packaging material with high barrier seal properties during dry ambient conditions, preferably below 15 % Rh.

The moisture-tight, high barrier seal containers, according to the present invention, which are loaded with metered doses of tiotropium should preferably be made out of aluminum foils approved to be in direct contact with pharmaceutical products. Aluminum foils that work properly in these aspects generally consist of technical polymers laminated with aluminum foil to give the foil the correct mechanical properties to avoid cracking of the aluminum during forming. Sealing of the formed containers is normally done by using a thinner cover foil of pure aluminum or laminated aluminum and polymer. The container and cover foils are then sealed together using at least one of several possible methods, for instance:
using a heat sealing lacquer, through pressure and heat;
using heat and pressure to fuse the materials together;
ultrasonic welding of the materials in contact.

Tiotropium in pure form is a potent drug and it is therefore diluted before a dose forming step by mixing with acceptable excipients, e.g. lactose, in selected ratio(s) in order to fit a preferred method of dose forming and loading. For example, details about inhalation powders containing tiotropium in mixtures with excipients, methods of powder manufacture, use of powder and capsules for powder may be studied in the international publication WO 02/30389 A1, Bechtold-Peters et al. Manufacturing a formulation of a very small amount of e.g. tiotropium with a much larger quantity of excipient requires special precautions to be taken to give a final, stable and robust manufacturing method.

According to the present invention a delivered fine particle dose (FPD) of pure tiotropium administered by inhalation herein is not limited, and may generally be in a range from 1 to 25 µg, including 5, 10, 15, and 20 µg. The selected dose size is usually prescribed by a physician and depends on the age, weight and gender of the patient as well as the severity of the medical condition. However, dry tiotropium powder exists normally as a chemical compound, a salt for example. Depending on the preferred chemical composition of the substance, such as tiotropium in the example, the dose mass usually is modified to give the corresponding effect of the intended dose of pure tiotropium. For instance, if tiotropium bromide monohydrate is to be used as the active ingredient the typical FPD falls in a range from 1.25 to 31.25 µg. Further, the correct metered dose loaded into an inhaler to be used for the purpose of administration must be adjusted for predicted losses such as retention and more or less efficient de-aggregation of the inhaled dose.

### Powder flow properties

The powder flow property of a formulation is important in establishing a robust production method using volumetric or gravimetric filling methods. Two properties are of major importance are:
Particle size
Particle surface
Excipient particles having a physical median particle size larger than 25 µm and having a very narrow particle size distribution with generally less than 5 % of the particles by mass being below 10 µm generally show good flow properties, and are particularly suitable for use in mixtures together with tiotropium. Large particles of excipients or APIs may act as carriers of small particles, in this case small particles of tiotropium: For inhalation purposes carrier particles having a mass median particle size in a range from 10 to 250 µm are typically selected, including 30, 50, 70, 100, 130, 160, 190, and 220 µm. The best median particle size chosen within this range depends on many factors, e.g. type of carrier substance, degree of powder flowability to be attained, type of inhaler and ease of de-aggregation during inhalation of the resulting medicament. Commercial grades of Respitos are available (lactose monohydrate from DMV of several defined particle size distributions up to 400 µm) suitable as particular excipients to be used in formulations containing tiotropium, e.g. grade SV003. Uniform homogeneous tiotropium powder formulations having a physical median particle size down to 10 µm can also provide good flow properties when the particles have been modified to have a very smooth surface, thereby improving the flow properties of the formulation. Laboratory tests show that up to 20 % of fine particles (w/w fine) of APIs, i.e. smaller than 10 µm, are possible to mix with larger particles, i.e. larger than 25 µm, and still maintain a stable formulation with very good FPD properties. Generally, large particles account for more than 80 % (w/w) of the dose mass when using volumetric dose forming methods.

A practical lower limit for volumetric dose forming is in a range 0.5 to 1 mg. Smaller doses are very difficult to produce and still maintain a low relative standard deviation between doses in the order of 10 %. Typically, though, dose masses are in a range from 1 to 10 mg.

Suitable excipients for inclusion in a tiotropium formulation include monosaccarides, disaccarides, polylactides, oligo- and polysaccarides, polyalcohols, polymers, salts or mixtures from these groups, e.g. glucose, arabinose, lactose, lactose monohydrate, lactose unhydrous [i.e., no crystalline water present in lactose molecule], saccharose, maltose, dextrane, sorbitol, mannitol, xylitol, sodium chloride, calcium carbonate. A particular excipient is lactose.

In our findings regarding the sensitivity to moisture for tiotropium powders the moisture properties of any proposed excipient must be appropriate before it is selected for inclusion in a formulation comprising tiotropium, regardless of the function of the proposed excipient. An excipient which, after dose forming, gives off much water inside the container enclosing the dose of mixed powders may negatively affect the included active powder, such that the resulting FPD deteriorates rapidly after dose forming. Therefore, excipients to be mixed with tiotropium must be selected primarily among acceptable excipients, which have good moisture qualities in the sense that the substance will not adversely affect the active medicament FPD for the shelf life of the product regardless of normal changes in ambient conditions during storage. Suitable "dry" excipients include those in the above-mentioned groups. In a preferred embodiment lactose is selected as the dry excipient and most preferably lactose monohydrate to be used in a mixture with tiotropium. One reason for selecting lactose as excipient is its inherent property of having a low and constant water sorption isotherm. Excipients having a similar or lower sorption isotherm may also be considered for use, provided other required qualities are met.

Ambient conditions during dose forming, loading and container sealing should be closely controlled. The temperature should preferably be below 25 □C and relative humidity should preferably be below 15 % Rh. The powder formulation should also be kept as dry as possible during the dose forming process. Taking these precautions will ensure that only a very small, acceptable amount of water is enclosed in the container together with the dose and not enough to present a threat to the stability of the moisture sensitive substance and the FPD. The original fine particle fraction (FPF) of the medicament dose (e.g. tiotropium) manifested in a high fine particle dose (FPD) of the metered dose of the medical product at the packaging stage is preserved in the high barrier seal container. Thus, when the pre-metered dose is delivered by a DPI it is unaffected for the shelf life of the medical product by normal variations in ambient conditions during handling, storage and delivery.

In a further aspect of the invention tiotropium may be mixed or formulated with one or more other pharmacologically active ingredient(s) (API), besides selected excipient(s), with an object of combining the anticholinergic agent with other medicament(s) to be used in a treatment of, e.g., respiratory disorders. The present invention encompasses such use of tiotropium where a combination of tiotropium with other medicaments constitute a formulation from which metered doses are then produced, filled and sealed into dry, moisture-tight, high barrier seal containers intended for insertion into a DPI to be administered according to a particular dosing regime or as needed by the user. In a particular embodiment at least one selected API may supplant one or more selected excipients, such that the sum of the tiotropium dose and the added API(s) satisfies all requirements regarding compatibility, moisture properties, FPD stability, potencies and total dose mass. Examples of interesting combinations of substances together with tiotropium include:
Inhaled steroids: E.g. budesonid, fluticasone, rofleponide, mometasone, ciclesonide.
Anti-histamines: E.g. epinastine, cetirizine, azelastine, fexofenadine, levocabastine, loratadine, mizolastine, ketotifene, emedastine, dirnetindene, clemastine, bamipine, cexchlorpheniramine, pheniramine, doxylamine, chlorphenoxamine, dimenhydrinate, diphenhydramine, promethazine, ebastine, desloratidine and meclozine.
Beta-mimetics: E.g. formoterol, salmeterol, salbutamol, terbutalinsulphate. PDE IV inhibitors: E.g. 3',5' - cyclic nucleotide phosphodiesterases and derivates.
Adenosine A2a receptor agonists: E.g. Ribofuranosylvanamide and derivates, substances described in publication WO 02/94273.
The sealed, dry, high barrier container of the invention that is directly loaded with a formulation of tiotropium may be in the form of a blister and it may e.g. comprise a flat dose bed or a formed cavity in aluminum foil or a molded cavity in a polymer material, using a high barrier seal foil against ingress of moisture, e.g. of aluminum or a combination of aluminum and polymer materials. The sealed, dry, high barrier container may form a part of an inhaler device or it may form a part of a separate item intended for insertion into an inhaler device for administration of doses. The sealed high barrier container used in the C-haler test described in the foregoing had the following data:
   - Container internal volume: 100 mm³
   - Effective diffusion area: 46 mm²
   - Diffusion constant: 0.044 g/m² for 24 hours at 23 °C and differential Rh = 50 %

Expressed in a different way, the diffusion of water into the container was in this case at a rate of 20 g/m³ per 24 hours at 23 °C at a presumed driving difference in Rh of 50 %. The results from the C-haler test show that the applied container was adequate in protecting the dose for 14 days. Thus, the present invention teaches that e.g. a sealed high barrier container of the size above holding a dose of tiotropium should not have a water transmission rate of more than 20 g/m³ for 24 hours at 23 °C and differential Rh = 50 % conditions to be suitable for an in-use time of maximum 2 weeks. The results from the C-haler test may be transposed into a set of demands put on a different type of container, e.g. a blister. A blister of similar size to the C-haler cartridge would have to be made using a typical high quality material like 50 µm PCTFE/PVC, which just meets the diffusion constant of the C-haler container (=0.118 g/m² when re-calculated to @38 °C and 90 % Rh). If a device with a container of tiotropium is intended to be in use for longer periods than 2 weeks, then a more moisture tight container must be used to protect the FPD.

Our tests indicate that compositions of tiotropium and at least one excipient and developed according to methods described in this application show exceptionally good FPD data and the compositions are stable over time and during in-use time if filled into high barrier seal containers.

In order to develop a formulation of tiotropium having controlled moisture properties a study into the chemical and physical properties of the chosen excipient should first be carried out. The sorption isotherm properties will give information with respect to how a formulation will respond to different temperatures and relative humidity in its surrounding environment. One very important question is also the "memory" of some excipients built in by the fact that it takes a very long time to reach steady state for the excipient after a disturbance in the environment. A suitable excipient for a formulation comprising tiotropium is an excipient like lactose monohydrate. The isotherm of lactose monohydrate has three important properties:
- Low absolute water content
- Low change in absolute water content after a change in relative humidity.
- Highly stable in in-use temperature situations

Low absolute water content ensures that a disturbance from steady conditions will not have a big impact on a tiotropium dose when the total amount of water present in the excipient is low. The low change in absolute water content at different relative humidity ensures that the excipient has no "memory" and that it can easily be put into a steady state at a given relative humidity before filling into a high barrier container. The temperature stability ensures that adsorption and desorption inside the high barrier seal will influence the API as little as possible.

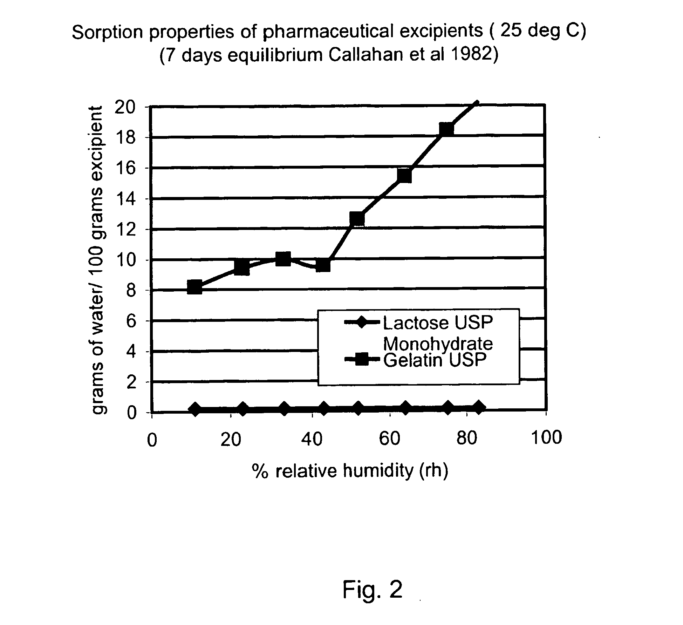

Fig 2 shows the isotherms of gelatine today used in the Spiriva^{®} product and lactose monohydrate as examples of a bad and a good choice of excipient or materials for a moisture sensitive tiotropium powder formulation. The effect of the excipient is normally very big when the amount of API is low. In using a volumetric dose forming method the formulation must possess certain physical flow properties making it necessary to add larger excipient particles into the formulation. For tiotropium in the form of the Spiriva^{®} formulation a relation between the API and the excipient or excipients is more than 1:250, which implies that a small variation in the excipient qualities, e.g. its moisture properties, may have an extremely big impact on the API and the performance of the formulation. If the electric field dosing technologies (ELFID) dose forming method is used the relationship between API and excipient or excipients may be limited to less than 1:10 making the impact of the excipient variation much less critical than for volumetric dose forming.

A good understanding of the above-described considerations in choosing suitable excipients is necessary to ensure that the formulation of the anticholinergic substance will not change in FPD if a dose of the formulation is loaded into a high barrier container, even if the container is subjected to big changes in the ambient climate.

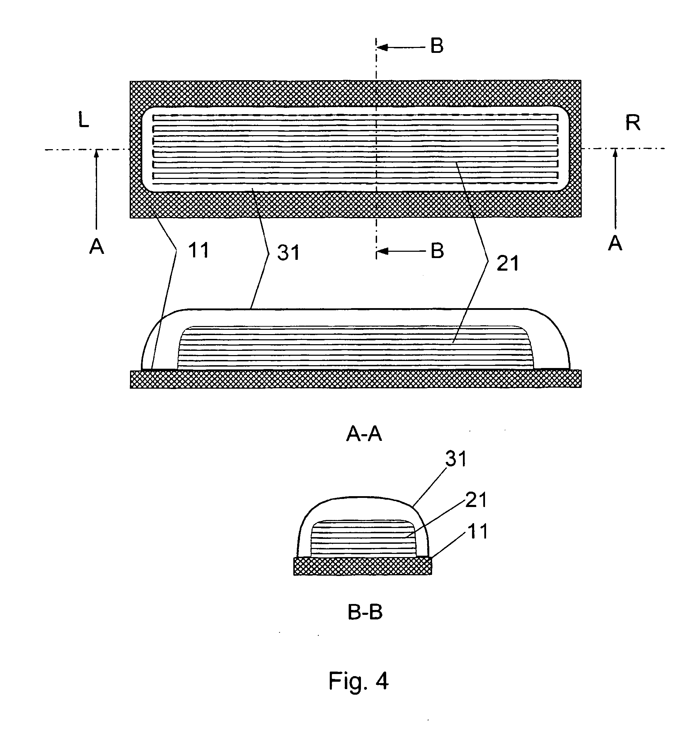

Thus, in order to develop a formulation of tiotropium offering the best possible FPD out of a pre-metered dry powder inhaler, a method to produce an optimal formulation of the API with the excipient must also be considered. See flow-chart illustrated in figure 4. Choosing tiotropium as an example of a very potent drug a first dilution must be made. The following method can be used:
1. In a first step, the minimum volumetric dose mass of the tiotropium formulation is determined. Normally in practice, the minimum dose mass is in a range from 1000 to 5000 µg, although recent, improved dose forming methods may safely specify a minimum dose mass below 500 µg. The dilution ratio follows as a result of the specified mass of tiotropium compound and the specified minimum dose mass.
2. **Alternative A; Uniform mixtures and blends of tiotropium powder formulation:**
   In a second step the tiotropium powder is diluted to have a correct minimum dose mass, as determined, preferably using a dry excipient having a physical particle size > 25 µm using a method that produces a uniform mixture. Preferably, this is made by dry mixing of the excipient and the tiotropium powders together, either in a continuous or batch process.
3. **Alternative B; Uniform homogeneous tiotropium powder formulation:**
   In a second step the tiotropium powder is diluted to have a correct minimum dose mass, as determined, using a dry excipient and feed the excipient as appropriate into the process that prepares homogeneous tiotropium particles. For example, this process may be spray drying or freeze-drying.

To protect the FPD up to the very point of aerosolizing of the dose a method of opening the dose container a fraction of a second before the dose starts to be aerosolized is presented and can be studied in detail in our publication WO 02/24266 A1, which is hereby included in this document in its entirety as a reference. In this context it is also important to prevent a voluntary or involuntary exhalation from a user of a DPI, who is about to inhale a dose, from reaching the selected dose, because of the high moisture content in the exhalation air. In our publication US 6,439,227 B1, which is hereby included in this document in its entirety as a reference, a device is disclosed, which closes the DPI, should the user exhale, so that exhalation air does not reach the dose container and the selected dose in the DPI. The device also controls the release of a cutter and a suction nozzle such that the cutter cannot open the container and inspiration air cannot begin to aerosolize the dose until a certain selected pressure drop is present due to a suction effort by the user.

The present invention teaches the importance of preventing moisturized air from a user or from ambient air from reaching the powder in the dose prior to an inhalation and stresses the importance of making the dose available for aerosolizing preferably in direct connection with the breaking of the seal to the container enclosing the dose. Preferably, the time period when the dose is exposed to ambient air, after breaking of the container seal, should not exceed 2 minutes, or else the FPD may drop when the dose is finally delivered, because tiotropium may be adversely affected by moisture in the ambient air, even if the powder is only exposed for a couple of minutes.

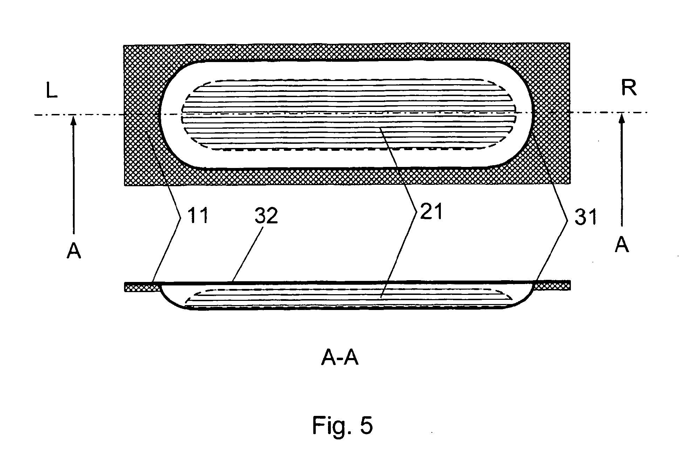

An inhaler providing a prolonged delivery of a dose from a high barrier seal container during the course of a single inhalation constitutes a preferred embodiment of an inhaler for the delivery of the tiotropium powder formulation. An Air-razor method as described in our publication US 2003/0192539 A1 is preferably applied in the inhaler to efficiently and gradually aerosolize the dose when delivered to the user. Surprisingly enough, applying an inhaler for a prolonged delivery and using the Air-razor method on a dose comprising tiotropium in Spiriva^{®} formulation results in an FPD at least twice as big as that from the state-of-the-art HandiHaler^{®} See examples of doses illustrated in figures 4 and 5.

In Figures 4 and 5 reference numbers **11 - 32** of the drawings like numbers indicate like elements throughout both views of two different embodiments of doses of a dry powder medicament comprising a tiotropium powder formulation loaded onto a dose bed of a container as illustrated, presented here as non-limiting examples.

Figure 4 illustrates a side and a top view of a dose **21** loaded onto a dose bed **11** of a high barrier container, the dose sealed moisture-tight by a high barrier seal **31.**

Figure 5 illustrates a side and a top view of a dose **21** loaded onto a dose bed **11** of a high barrier container, the dose sealed moisture-tight by a high barrier seal **31** and **32.**

As used herein, the phrases "selected from the group consisting of," "chosen from," and the like include mixtures of the specified materials. All references, patents, applications, tests, standards, documents, publications, brochures, texts, articles, instructions, etc. mentioned herein are incorporated herein by reference. Where a numerical limit or range is stated, the endpoints are included. Also, all values and sub-ranges within a numerical limit or range are specifically included as if explicitly written out.

The above written description of the invention provides a manner and process of making and using it such that any person skilled in this art is enabled to make and use the same, this enablement being provided in particular for the subject matter of the appended claims, which make up a part of the original description, and including the following inventive concepts: a pre-metered dry powder inhaler, comprising a dry powder medicament dose and a container, wherein the dry powder medicament dose is loaded into said container and comprises particles of tiotropium and particles of at least one dry excipient, the container constitutes a dry, high barrier seal, whereby the high barrier seal of the container prevents ingress of moisture thereby preserving the dry powder medicament dose, and the dry powder medicament dose in the container has been formed by either volumetric or electric field dose forming methods; the at least one dry excipient is present in the medicament dose as finely divided particles having a diameter of 10 µm or more, and the at least one dry excipient comprises an excipient selected from the group consisting of monosaccarides, disaccarides, polylactides, oligo- and polysaccarides, polyalcohols, polymers, salts and mixtures thereof; the at least one dry excipient is present in the medicament dose as particles having a diameter of 25 □m or more in an amount of more than 80 % by weight, and the at least one dry excipient comprises an excipient selected from the group consisting of monosaccarides, disaccarides, polylactides, oligo- and polysaccarides, polyalcohols, polymers, salts and mixtures thereof; the dry, high barrier seal is formed of a material selected from the group consisting of metals, thermoplastics, glass, silicon, silicon oxides and mixtures thereof; the inhaler is adapted such that administration of the dry powder dose is performed by inhalation from a dry powder inhaler providing a prolonged dose delivery; the excipient is selected from the group consisting of lactose, lactose unhydrous, lactose monohydrate and mixtures thereof; the dry, high barrier seal comprises flat aluminum foils, optionally laminated with one or more polymers;the container forms a cavity molded from a polymer material selected to give the container high barrier seal properties; the container forms a cavity molded from a polymer material together with a high barrier seal providing it with high barrier seal properties; the container is a part of a dry powder inhaler; the container is a separate part adapted for insertion into a dry powder inhaler; the container is a separate part comprising a primary part adapted for insertion into a dry powder inhaler and a secondary part enclosing the primary part in a moisture-tight package; the dry powder medicament dose is for use in a treatment of a respiratory disorder; the high barrier seal consists of peelable foils; the high barrier seal is a rigid unitary magazine including a plurality of integral reservoirs; the high barrier seal is a compartment having a first and a second face sealed with foils, said foils being capable of being ruptured before inhalation; the dose of the medicament delivered from a dry powder inhaler represents more than 20 % of the pre-metered dose and 40 % of the delivered dose; the dry powder medicament dose further comprises at least one additional active pharmaceutical ingredient selected from the group consisting of inhalable steroids, nicotinamide derivatives, beta-agonists, beta-mimetics, anti-histamines, adenosine A2A receptors, PDE4 inhibitors, dopamine D2 receptor agonists, and mixtures thereof; the at least one second additional pharmaceutical ingredient is selected from the group consisting of budesonid, fluticasone, rofleponide, mometasone, ciclesonide epinastine, cetirizine, azelastine, fexofenadine, levocabastine, loratadine, mizolastine, ketotifene, emedastine, dirnetindene, clemastine, bamipine, cexchlorpheniramine, pheniramine, doxylamine, chlorphenoxamine, dimenhydrinate, diphenhydramine, promethazine, ebastine, desloratidine, meclozine, formoterol, salmeterol, salbutamol, terbutalinsulphate, 3',5' - cyclic nucleotide phosphodiesterases and derivates, ribofuranosylvanamide and mixtures thereof; and a dry powder medicament dose loaded into a container and formed by either volumetric or electric field dose forming methods, said dose comprising particles of tiotropium and particles of at least one dry excipient, wherein the container constitutes a dry, high barrier seal preventing ingress of moisture and thereby preserving the dry powder medicament dose.

As is clear from the above specification, another particular embodiment of the invention is a dry powder inhaler comprising a dry powder medicament dose loaded into a container adapted for use in the dry powder inhaler, wherein the dry powder medicament dose comprises: particles of tiotropium; and particles of at least one dry excipient; and wherein the container constitutes a dry, high barrier seal preventing ingress of moisture and preserving the dry powder medicament dose. In one particular embodiment, the medicament dose is kept dry by the container such that, for example, the original FPD at the filling stage is maintained for example at 40 C and 75% Rh for 14 days. Alternatively, or additionally, the sealed high barrier-comprising container of the invention preferably does not have a water transmission rate of more than 20 g/m³ for 24 hours at 23 °C and differential Rh = 50 %. Alternatively, or additionally, the sealed high barrier-comprising container of the invention does not affect the tiotropium FPD - e.g., a consistent FPD is maintained, over the expected shelf life of the product.

## Claims

1. A pre-metered dry powder inhaler, comprising a dry powder medicament dose and a container, **characterized in that**
the dry powder medicament dose is loaded into said container and comprises particles of tiotropium and particles of at least one dry excipient; the container constitutes a dry, high barrier seal, comprising aluminium whereby the high barrier seal of the container prevents ingress of moisture thereby preserving the dry powder medicament dose; and the dry powder medicament dose in the container has been formed by either volumetric or electric field dose forming methods.

2. The pre-metered dry powder inhaler according to claim 1, **characterized in that** the at least one dry excipient is present in the medicament dose as finely divided particles having a diameter of 10 pm or more; and the at least one dry excipient comprises an excipient selected from the group consisting of monosaccarides, disaccarides, polylactides, oligo-and polysaccarides, polyalcohols, polymers, salts and mixtures thereof.

3. The pre-metered dry powder inhaler according to claim 1, **characterized in that** the at least one dry excipient is present in the medicament dose as particles having a diameter of 25 µm or more in an amount of more than 80 % by weight; and the at least one dry excipient comprises an excipient selected from the group consisting of monosaccarides, disaccarides, polylactides, oligo-and polysaccarides, polyalcohols, polymers, salts and mixtures thereof

4. The pre-metered dry powder inhaler according to claim 1, **characterized in that** the inhaler is adapted such that administration of the dry powder dose is performed by inhalation from a dry powder inhaler providing a prolonged dose delivery.

5. The pre-metered dry powder inhaler according to claim 1, **characterized in that** the excipient is selected from the group consisting of lactose, lactose anhydrous, lactose monohydrate and mixtures thereof.

6. The pre-metered dry powder inhaler according to claim 1, **characterized in that** the dry, high barrier seal comprises flat aluminum foils, optionally laminated with one or more polymers.

7. The pre-metered dry powder inhaler according to claim 1, **characterized in that** the container forms a cavity molded from a polymer material selected to give the container high barrier seal properties.

8. The pre-metered dry powder inhaler according to claim 1, **characterized in that** the container forms a cavity molded from a polymer material together with a high barrier seal providing it with high barrier seal properties.

9. The pre-metered dry powder inhaler according to claim 1, **characterized in that** the container is a part of said dry powder inhaler.

10. The pre-metered dry powder inhaler according to claim 1, **characterized in that** the container is a separate part adapted for insertion into said dry powder inhaler.

11. The pre-metered dry powder inhaler according to claim 1, **characterized in that** the container is a separate part comprising a primary part adapted for insertion into said dry powder inhaler and a secondary part enclosing the primary part in a moisture-tight package.

12. The pre-metered dry powder inhaler according to claim 1, **characterized in that** the high barrier seal consists of peelable foils.

13. The pre-metered dry powder inhaler according to claim 1, **characterized in that** the high barrier seal is a rigid unitary magazine including a plurality of integral reservoirs.

14. The pre-metered dry powder inhaler according to claim 1, **characterized in that** the high barrier seal is a compartment having a first and a second face sealed with foils, said foils being capable of being ruptured before inhalation.

15. The pre-metered dry powder inhaler according to claim 1, **characterized in that** the dose of the medicament delivered from said dry powder inhaler represents more than 20 % of the pre-metered dose and 40 % of the delivered dose.

16. The pre-metered dry powder inhaler according to claim 1, **characterized in that** said dry powder medicament dose further comprises at least one additional active pharmaceutical ingredient selected from a group consisting of inhalable steroids, nicotinamide derivatives, beta-agonists, beta-mimetics, anti-histamines, adenosine A2A receptors, PDE4 inhibitors, dopamine D2 receptor agonists, and mixtures thereof.

17. The pre-metered dry powder inhaler according to claim 16, **characterized in that** the at least one second additional pharmaceutical ingredient is selected from a group consisting of budesonid, fluticasone, rofleponide, mometasone, ciclesonide epinastine, cetirizine, azelastine, fexofenadine, levocabastine, loratadine, mizolastine, ketotifene, emedastine, dimetindene, clemastine, bamipine, cexchlorpheniramine, pheniramine, doxylamine, chlorphenoxamine, dimenhydrinate, diphenhydramine, promethazine, ebastine, desloratidine, meclozine, formoterol, salmeterol, salbutamol, terbutalinsulphate, 3', 5'-cyclic nucleotide phosphodiesterases and derivates, ribofuranosylvanamide and mixtures thereof.

18. A dry powder medicament dose loaded into a container and formed by either volumetric or electric field dose forming methods, said dose comprising particles of tiotropium and particles of at least one dry excipient, **characterized in that** the container constitutes a dry, high barrier seal comprising aluminium preventing ingress of moisture and thereby preserving the dry powder medicament dose.

## Patentansprüche

1. Vordosierter Trockenpulverinhalator, umfassend eine Dosis eines Trockenpulverarzneimittels und einen Behälter, **dadurch gekennzeichnet, dass** die Trockenpulverarzneimitteldosis in den Behälter gefüllt wird und Partikel von Tiotropium und Partikel von mindestens einem trockenen Hilfsstoff umfasst; der Behälter eine trockene hochwirksame Abdichtung bzw. Dichtung aufweist, die Aluminium umfasst, wobei die hochwirksame Abdichtung bzw. Dichtung des Behälters das Eindringen von Feuchtigkeit verhindert, wodurch die Trockenpulverarzneimitteldosis geschützt wird; und die Trockenpulverarzneimitteldosis im Behälter durch entweder volumetrische Dosisbildungsverfahren oder Dosisbildungsverfahren durch ein elektrisches Feld gebildet wurde.

2. Vordosierter Trockenpulverinhalator nach Anspruch 1, **dadurch gekennzeichnet, dass** der mindestens eine trockene Hilfsstoff in der Arzneimitteldosis in Form fein verteilter Partikel mit einem Durchmesser von 10 pm oder mehr vorliegt; und der mindestens eine trockene Hilfsstoff einen Hilfsstoff umfasst, ausgewählt aus der Gruppe, bestehend aus Monosacchariden, Disacchariden, Polylactiden, Oligo- und Polysacchariden, Polyalkoholen, Polymeren, Salzen und Mischungen hiervon.

3. Vordosierter Trockenpulverinhalator nach Anspruch 1, **dadurch gekennzeichnet, dass** der mindestens eine trockene Hilfsstoff in der Arzneimitteldosis in Form von Partikeln mit einem Durchmesser von 25 µm oder mehr in einer Menge von mehr als 80 Gew.-% vorliegt; und der mindestens eine trockene Hilfsstoff einen Hilfsstoff umfasst, ausgewählt aus der Gruppe, bestehend aus Monosacchariden, Disacchariden, Polylactiden, Oligo- und Polysacchariden, Polyalkoholen, Polymeren, Salzen und Mischungen hiervon,

4. Vordosierter Trockenpulverinhalator nach Anspruch 1, **dadurch gekennzeichnet, dass** der Inhalator so angepasst ist, dass die Verabreichung der Trockenpulverdosis durch Inhalation aus einem Trockenpulverinhalator erfolgt, der eine verlängerte Dosisabgabe bereitstellt.

5. Vordosierter Trockenpulverinhalator nach Anspruch 1, **dadurch gekennzeichnet, dass** der Hilfsstoff ausgewählt ist aus der Gruppe, bestehend aus Lactose, wasserfreier Lactose, Lactosemonohydrat und Mischungen hiervon.

6. Vordosierter Trockenpulverinhalator nach Anspruch 1, **dadurch gekennzeichnet, dass** die trockene hochwirksame Abdichtung bzw. Dichtung flache Aluminiumfolien aufweist, die gegebenenfalls mit einem oder mehreren Polymeren laminiert sind.

7. Vordosierter Trockenpulverinhalator nach Anspruch 1, **dadurch gekennzeichnet, dass** der Behälter einen Hohlraum bildet, geformt aus einem Polymermaterial, ausgewählt, um dem Behälter hochwirksame Abdichtungseigenschaften zu verleihen.

8. Vordosierter Trockenpulverinhalator nach Anspruch 1, **dadurch gekennzeichnet, dass** der Behälter einen Hohlraum bildet, geformt aus einem Polymermaterial, zusammen mit einer hochwirksamen Abdichtung bzw. Dichtung, was ihm hochwirksame Abdichtungseigenschaften verleiht.

9. Vordosierter Trockenpulverinhalator nach Anspruch 1, **dadurch gekennzeichnet, dass** der Behälter einen Teil des Trockenpulverinhalators darstellt.

10. Vordosierter Trockenpulverinhalator nach Anspruch 1, **dadurch gekennzeichnet, dass** der Behälter einen getrennten Teil darstellt, dafür angepasst, um in den Trockenpulverinhalator eingesetzt zu werden.

11. Vordosierter Trockenpulverinhalator nach Anspruch 1, **dadurch gekennzeichnet, dass** der Behälter einen getrennten Teil darstellt, umfassend einen ersten Teil, angepasst, um in den Trockenpulverinhalator eingesetzt zu werden, und einen zweiten Teil, der den ersten Teil in einer feuchtigkeitsdichten Einheit um- bzw. einschließt.

12. Vordosierter Trockenpulverinhalator nach Anspruch 1, **dadurch gekennzeichnet, dass** die hochwirksame Abdichtung bzw. Dichtung aus ablösbaren bzw. abziehbaren Folien besteht.

13. Vordosierter Trockenpulverinhalator nach Anspruch 1, **dadurch gekennzeichnet, dass** die hochwirksame Abdichtung bzw. Dichtung ein starres einheitliches Magazin, enthaltend eine Vielzahl von integrierten Reservoirs, darstellt.

14. Vordosierter Trockenpulverinhalator nach Anspruch 1, **dadurch gekennzeichnet, dass** die hochwirksame Abdichtung bzw. Dichtung eine Kammer darstellt, mit einer ersten und einer zweiten Seite, abgedichtet mit Folien, wobei die Folien vor der Inhalation abgerissen bzw. zerstört werden können.

15. Vordosierter Trockenpulverinhalator nach Anspruch 1, **dadurch gekennzeichnet, dass** die Dosis des Arzneimittels, das vom Trockenpulverinhalator zugeführt wird, mehr als 20% der vordosierten Dosis und 40% der zugeführten Dosis darstellt.

16. Vordosierter Trockenpulverinhalator nach Anspruch 1, **dadurch gekennzeichnet, dass** die Trockenpulverarzneimitteldosis weiterhin mindestens einen zusätzlichen pharmazeutisch aktiven Bestandteil aufweist, ausgewählt aus einer Gruppe, bestehend aus inhalierbaren Steroiden, Nicotinamidderivaten, beta-Agonisten, beta-Mimetika, Antihistaminen, Adenosin-A2A-Rezeptoren, PDE4-Inhibitoren, Dopamin-D2-Rezeptor-agonisten und Mischungen hiervon.

17. Vordosierter Trockenpulverinhalator nach Anspruch 16, **dadurch gekennzeichnet, dass** mindestens ein zweiter zusätzlicher pharmazeutischer Bestandteil ausgewählt ist aus einer Gruppe, bestehend aus Budesonid, Fluticason, Rofleponid, Mometason, Ciclesonid, Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin, Meclozin, Formoterol, Salmeterol, Salbutamol, Terbutalinsulfat, 3',5'-zyklische Nucleotidphosphodiesterasen und Derivate, Ribofuranosylvanamid und Mischungen hiervon.

18. Trockenpulverarzneimitteldosis, gefüllt in einen Behälter und gebildet durch volumetrische Dosisbildungsverfahren oder Dosisbildungsverfahren durch ein elektrisches Feld, wobei die Dosis Partikel von Tiotropium umfasst und Partikel von mindestens einem trockenen Hilfsstoff, **dadurch gekennzeichnet, dass** der Behälter eine trockene hochwirksame Abdichtung bzw. Dichtung, umfassend Aluminium, aufweist, was dem Eindringen von Feuchtigkeit vorbeugt, und hierdurch die Trockenpulverarzneimitteldosis schützt.

## Revendications

1. Inhalateur de poudre sèche pré-dosé, comprenant une dose de médicament sous forme de poudre sèche, et un récipient, **caractérisé en ce que** le médicament sous forme de poudre sèche est chargé dans ledit récipient et comprend des particules de tiotropium et des particules d'au moins un excipient sec ; le récipient constitue une barrière d'étanchéité élevée sèche, comprenant de l'aluminium, moyennant quoi la barrière d'étanchéité élevée du récipient empêche l'entrée d'humidité, en préservant ainsi la dose de médicament sous forme de poudre sèche ; et la dose de médicament sous forme de poudre sèche présente dans le récipient a été formée par des procédés de formation de la dose par champ électrique ou volumétrique.

2. Inhalateur de poudre sèche pré-dosé selon la revendication 1, **caractérisé en ce que** ledit au moins un excipient sec est présent dans la dose de médicament sous la forme de particules finement divisées ayant un diamètre de 10 pm ou plus ; et ledit au moins un excipient sec comprend un excipient choisi dans le groupe constitué des monosaccharides, des disaccharides, des polylactides, des oligo- et polysaccharides, des polyalcools, des polymères, des sels et des mélanges de ceux-ci.

3. Inhalateur de poudre sèche pré-dosé selon la revendication 1, **caractérisé en ce que** ledit au moins un excipient sec est présent dans la dose de médicament sous forme de particules ayant un diamètre de 25 µm ou plus, dans une quantité de plus de 80% en poids, et ledit au moins un excipient sec comprend un excipient choisi dans le groupe constitué des monosaccharides, des disaccharides, des polylactides, des oligo- et polysaccharides, des polyalcools, des polymères, des sels et des mélanges de ceux-ci.

4. Inhalateur de poudre sèche pré-dosé selon la revendication 1, **caractérisé en ce que** l'inhalateur est adapté à ce que l'administration de la dose de poudre sèche soit effectuée par inhalation, à partir d'un inhalateur de poudre sèche fournissant une distribution de dose prolongée.

5. Inhalateur de poudre sèche pré-dosé selon la revendication 1, **caractérisé en ce que** l'excipient est choisi dans le groupe constitué du lactose, du lactose anhydre, du monohydrate de lactose et des mélanges de ceux-ci.

6. Inhalateur de poudre sèche pré-dosé selon la revendication 1, **caractérisé en ce que** la barrière d'étanchéité élevée sèche comprend des feuilles d'aluminium planes, éventuellement stratifiées avec un ou plusieurs polymères.

7. Inhalateur de poudre sèche pré-dosé selon la revendication 1, **caractérisé en ce que** le récipient forme une cavité moulée, à partir d'un matériau polymère choisi pour donner au récipient des propriétés de barrière d'étanchéité élevée.

8. Inhalateur de poudre sèche pré-dosé selon la revendication 1, **caractérisé en ce que** le récipient forme une cavité moulée à partir d'un matériau polymère avec une barrière d'étanchéité élevée lui fournissant des propriétés de barrière d'étanchéité élevée.

9. Inhalateur de poudre sèche pré-dosé selon la revendication 1, **caractérisé en ce que** le récipient fait partie dudit inhalateur de poudre sèche.

10. Inhalateur de poudre sèche pré-dosé selon la revendication 1, **caractérisé en ce que** le récipient est une partie séparée adaptée pour insertion dans ledit inhalateur de poudre sèche.

11. Inhalateur de poudre sèche pré-dosé selon la revendication 1, **caractérisé en ce que** le récipient est une partie séparée comprenant une partie primaire, adaptée pour insertion dans ledit inhalateur de poudre sèche et une partie secondaire, qui inclut la partie primaire dans un emballage étanche à l'humidité.

12. Inhalateur de poudre sèche pré-dosé selon la revendication 1, **caractérisé en ce que** la barrière d'étanchéité élevée est constituée de feuilles pelables.

13. Inhalateur de poudre sèche pré-dosé selon la revendication 1, **caractérisé en ce que** la barrière d'étanchéité élevée est un magasin unitaire rigide comprenant une pluralité de réservoirs d'un seul tenant.

14. Inhalateur de poudre sèche pré-dosé selon la revendication 1, **caractérisé en ce que** la barrière d'étanchéité élevée est un compartiment ayant une première et une seconde faces scellées avec des feuilles, lesdites feuilles pouvant être cassées avant inhalation.

15. Inhalateur de poudre sèche pré-dosé selon la revendication 1, **caractérisé en ce que** la dose de médicament distribuée par ledit inhalateur de poudre sèche représente plus de 20% de la dose pré-dosée et 40% de la dose distribuée.

16. Inhalateur de poudre sèche pré-dosé selon la revendication 1, **caractérisé en ce que** ladite dose de médicament sous forme de poudre sèche comprend en outre au moins un ingrédient pharmaceutique actif supplémentaire, choisi dans un groupe constitué des stéroïdes inhalables, des dérivés de nicotinamide, des bêta-agonistes, des bêtamimétiques, des antihistaminiques, des récepteurs d'adénosine A2A, des inhibiteurs de PDE4, des agonistes de récepteur de dopamine D2 et des mélanges de ceux-ci.

17. Inhalateur de poudre sèche pré-dosé selon la revendication 16, **caractérisé en ce que** ledit au moins un second ingrédient pharmaceutique additionnel est choisi dans le groupe constitué des budésonid, fluticasone, rofléponide, mométasone, ciclésonide épinastine, cétirizine, azélastine, fexofénadine, lévocabastine, loratadine, mizolastine, kétotifène, émédastine, dimétindène, clémastine, bamipine, cexchlorphéniramine, phéniramine, doxylamine, chlorphénoxamine, diménhydrinate, diphénhydramine, prométhazine, ébastine, desloratidine, meclozine, formotérol, salmétérol, salbutamol, terbutalinsulfate, phosphodiestérases de nucléotide 3',5'-cyclique et leurs dérivés, ribofuranosylvanamide et des mélanges de ceux-ci.

18. Dose de médicament sous forme de poudre sèche chargé dans un récipient et formé par des procédés de formation de dose par champ électrique ou volumétrique, ladite dose comprenant des particules de tiotropium et des particules d'au moins un excipient sec, **caractérisée en ce que** le récipient constitue une barrière d'étanchéité élevée sèche, comprenant de l'aluminium, empêchant l'entrée d'humidité et préservant ainsi la dose de médicament sous forme de poudre sèche.
